# NEUE EUROPÄISCHE PATENTSCHRIFT

(11) **EP 0 211 166 B2**
(45) Veröffentlichungstag und Bekanntmachung des Hinweises auf die Entscheidung über den Einspruch: **01.09.1993**
(45) Hinweis auf die Patenterteilung: 03.01.1990
(21) Anmeldenummer: 86106941.7
(22) Anmeldetag: 22.05.1986
(51) Int. Cl.: A61N 1/05, A61N 1/39

(54) **Defibrillations-Elektrode**
Defibrillation electrode
Electrode de défibrillation

(30) Priorität: 28.06.1985 DE 3523226
(43) Veröffentlichungstag der Anmeldung: 25.02.1987
(73) Patentinhaber: Osypka, Peter, Dr. Ing., 79639 Grenzach-Wyhlen (DE)
(72) Erfinder: Osypka, Peter, Dr. Ing., 79639 Grenzach-Wyhlen (DE)
(74) Vertreter: Patent- und Rechtsanwaltssozietät, Schmitt, Maucher & Börjes

(56) Entgegenhaltungen:
- EP-A- 0 083 674
- EP-A- 0 095 726
- EP-A- 0 095 727
- EP-A- 0 134 367
- EP-A- 0 206 248
- WO-A-82/02663
- WO-A-82/02664
- DE-A- 2 516 712
- DE-A- 2 737 787
- FR-A- 2 299 873
- US-A- 3 244 174
- US-A- 4 030 509
- US-A- 4 112 952
- US-A- 4 144 889
- US-A- 4 444 207

## Beschreibung

Die Erfindung betrifft eine gegebenenfalls implantierbare Defibrillations-Elektrode mit einem biegsamen, vergrößerten Elektrodenkopf zum großflächigen Anlegen an beliebiger Stelle der Außenseite des Herzens und mit einer Zuleitung zur Verbindung des Elektrodenkopfes mit einem Defibrillator oder Impulsgenerator.

Für die temporäre und auch für die permanente elektrische Stimulation eines Herzens gibt es eine Vielzahl von Elektroden mit unterschiedlicher Polzahl. In der Regel werden die Elektroden transvenös eingeführt. Bekannt sind auch myokardiale Elektroden, die von außen auf das Herz aufgenäht oder aufgeschraubt werden. Die elektrische Energie zur Stimulation des Herzens besteht in der Regel aus einer Impuls-Folgefrequenz von etwa 70 Impulse pro Minute bei einer Spannung von 5 bis 10 Volt.

Eine dramatische Ausnahme, bei welcher die elektrische Stimulation des Herzens nicht mehr wirksam ist, bildet das Herzkammerflimmern.

Zur Beseitigung des Herzkammerflimmerns bedient man sich der elektrischen Defibrillation. Die elektrophysiologischen Verhältnisse bei der elektrischen Defibrillation sind sehr komplex. Ein normaler Herzschrittmacher-Impuls braucht im intakten Myokard nur eine einzige Stelle zu reizen.

Der Reiz breitet sich dann von selbst über das Reizleitungssystem des Herzens auf das ganze Herz aus. Beim flimmernden Herzen erfolgen völlig unkontrollierte kreisende Erregungswellen, die asynchron arbeiten. Dadurch kommt eine Pumpwirkung des Herzens nicht mehr zustande und der Patient stirbt, falls er nicht defibrilliert wird, d. h. falls nicht ein defibrillierender Stromstoß auf das Herz gegeben wird.

Ein defibrillierender Stromstoß mit einer Energie zwischen 200 bis 400 Joule depolarisiert alle im Herzen erregbaren Zellen, so daß danach wieder ein geordneter Ablauf der Kontraktion erfolgen kann. Zur Applikation des Stromstoßes benutz man großfläche, von außen auf den Thorax anlegbare Elektroden. Damit alle Zellen des Herzens depolarisiert werden können, muß die Energie der elektrischen Entladung so hoch gewählt werden, daß die elektrische Feldstärke an allen Abschnitten des Herzens mindest so groß ist, daß eine Depolarisation erfolgen kann.

Myokardgeschädigte Patienten sind hochgradig flimmergefährdet. Bei derartigen Patienten versucht man eine Abhilfe mit einem implantierbaren Defibrillator, der selbsttätig beim Beginn des Kammerflimmerns einen elektrischen Defibrillationsstoß über eine Defibrillations-Elektrode der eingangs erwähnten Art an das Herz abgibt. Zur Applikation des Stromstoßes verwendet man dabei in der Regel Elektroden mit großflächigen, maschenartigen Elektrodenköpfen, die auf den einander gegenüberliegenden Seiten des Herzens befestigt bzw. aufgenäht werden.

In ungünstiger Weise muß bei diesen Elektrodenköpfen eine relativ große Energie aufgebracht werden, damit alle erregbaren Zellen des Herzens depolarisiert werden. Hinzu kommt, daß der elektrische Kontakt zwischen Elektrodenkopf und Herzoberfläche wegen der Eigensteifigkeit der Elektrode nicht immer vollständig gewährleistet ist. Weiterhin wird das Herz selbst durch diese Steifigkeit des Elektrodenkopfes in seiner elastischen Ausdehnung behindert und damit in seiner Leistungsfähigkeit eingeschränkt.

Es besteht deshalb die Aufgabe, eine implantierbare Defibrillations-Elektrode der eingangs erwähnten Art zu schaffen welche weniger Energie für den DefibrillationsStromstoß benötigt, gleichzeitig dem Herzen eine bessere Bewegungsfreiheit läßt und eine Vielzahl von Anpassungsmöglichkeiten an die Anatomie der Herzoberfläche ergibt.

Diese scheinbar widersprüchliche Aufgabe wird dadurch gelöst, daß der Elektrodenkopf mehrere einzelne Elektrodenarme aufweist, die in Gebrauchsstellung stern- oder strahlenförmig auseinanderlaufen, einzeln an der Herzaußenseite befestigbar und allseitig Schwenkbar sind. Durch die Flexibilität der einzelnen Elektrodenarme wird das Herz in seiner Bewegung nur minimal beeinträchtigt. Dennoch ergibt sich eine gute Bedeckung der Herzoberfläche insbesondere, wenn die Elektrodenarme entsprechend groß und auch in großer Anzahl vorhanden sind, so daß die notwendige Energie zur Depolarisation der einzelnen Zellen reduziert werden kann, zumal die erfindungsgemäße Ausgestaltung des Elektrodenkopfes auch das Anbringen mehrerer derartiger Elektrodenköpfe ermöglicht, bei welchen gegebenenfalls die einzelnen Arme jeweils in die Zwischenräume zwischen den Armen des nächsten Elektrodenkopfes reichen könnten.

Für ein schnelles und dennoch wirkungsvolles Befestigen des Elektrodenkopfes ist es zweckmäßig, wenn die Befestigungselemente für die Elektrodenarme jeweils an deren freiem Ende etwa in der Verlängerung dieser Arme angeordnet und als Zug- oder Spannelemente ausgebildet sind. Beispiels-weise kann am freien Ende eines jeden Elektrodenarmes ein vorzugsweise elektrisch nicht leitender Faden und an dessen freiem Ende eine chirurgische Nadel zum Einziehen des Fadens und/oder des Elektrodenarmes in das Herzgewebe vorgesehen sein. Die Nadel und der wieder aus dem Herzgewebe austretende Teil des Fadens können anschließend abgeschnitten werden. Stattdessen oder zusätzlich kann am Ende des Elektrodenarmes ein quer zu dessen Erstreckung gegen das Innere des Herzens gerichteter Sporn oder Haken angreifen, mit welchen der einzelne in sich flexible Arm sehr einfach und schnell an der Herzaußenseite verankert werden kann, insbesondere dann, wenn dieser Sporn oder Haken mit seinem freien Ende etwas schräg von dem Armende gegen dessen Anfang hin weist, so daß die auftretenden Zugkräfte diesen Haken oder Sporn selbsttätig tiefer in das Herzgewebe ziehen

Dies geht umso besser und einfacher, wenn der Elektrodenarm als aus blankem Draht bestehende Wendel ausgebildet ist, die praktisch wie eine Zugfeder etwas unter Zugspannung gesetzt werden kann.

Gegebenenfalls kann der Elektrodenarm zur besseren Übertragung von Kräften und Energien als Mehrfachwendel, beispielsweise als Vierfachwendel ausgebildet sein.

Eine vorteilhafte Ausgestaltung der Erfindung kann darin bestehen, daß die den Elektrodenarm bildende Wendel ein im wesentlichen undehnbares Sicherheitsband zur Begrenzung oder Unterbindung einer Dehnung der Wendel in ihrer Längsrichtung aufweist. Dadurch bleibt die Wendel hochflexibel, um sich gut an die Herzbewegung anpassen zu können, jedoch wird eine Lockerung oder gar ein Ablösen aufgrund von Längsdehnungen vermieden. Auch kann beim Anlegen der einzelnen Elektrodenarme die Wendel nicht versehentlich zu sehr gedehnt werden. Das Sicherheitsband kann beispielsweise aus Metall und/oder einem Faden aus chirurgischem od. dgl. körperverträglichem Nahtmaterial bestehen.

Eine besonders zweckmäßige Ausgestaltung der Erfindung, die die Elektrode gegen Bewegungen des Patienten und des Herzens weitestgehend unempfindlich macht und die Reizübertragung verbessern kann, kann darin bestehen, daß der Elektrodenkopf zusätzlich zu seiner Befestigung mittels der Elektrodenarme auch an dem gemeinsamen Ausgangspunkt der Elektrodenarme - an der Herzaußenseite befestigbar oder verankerbar ist, insbesondere eine Befestigungsstelle oder Befestigungsvorrichtung, vorzugsweise eine zum Beispiel wendelförmige Schraube, Klammer od. dgl. aufweist.

Somit läßt sich auch das Zentrum der Elektrodenarme sicher festlegen, in dem es beispielsweise mit seiner Befestigungsstelle angenäht, eingeschraubt oder verhakt wird. Dadurch werden die Elektrodenarme beidseitig am Herzen festgelegt, so daß bei einer Festlegung unter einer Zugspannung, wie sie mit den vorbeschriebenen Merkmalen und Maßnahmen möglich ist, der Elektrodenarm auf seine ganze Länge wirksam wird und sich auch durch die Herzbewegung nicht lockert insbesondere dann, Wenn er sogar teilweise in die Herz-Oberfläche eingezogen ist.

Eine zweckmäßige Ausführungsform der Erfindung kann darin bestehen, daß die Elektrodenzuleitung - vorzugsweise an der Oberseite - an einer Halteplatte mündet oder endet, von deren Unterseite ein Einschraubwendel od. dgl. und/oder die Elektrodenarme ausgehen.

Die Elektrodenarme können gegenüber ihrer Halterung schwenkbar sein und vor dem Anlegen der Elektrode mit ihren Enden und ihren Halterungen an der Zuleitung der Elektrode anliegen. Dadurch läßt sich die gesamte Elektrode und insbesondere ihr Elektrodenkopf auch durch relativ enge Öffnungen implantieren, obwohl der Elektrodenkopf in Gebrauchsstellung durch die dann ausgebreiteten Arme eine große Fläche des Herzens beaufschlagen kann.

Eine Abwandlung oderweitere Ausgestaltung der Erfindung kann darin bestehen, daß die Elektrodenarme mit einem Draconnetz verbunden sind, welches am Herzen annähbar ist. Ein solches Netz ist sehrflexibel, erlaubt aber dennoch eine großflächige und sichere Befestigung der Elektrodenarme auch zwischen ihren Enden.

Ferner ist es möglich, daß die Elektrodenarme von ihrer Halterung abtrennbar sind. Unter Umständen kann es nämlich zweckmäßig sein, nur einzelne der am Elektrodenkopf vorgesehenen Elektrodenarme zu verwenden. Die nicht benötigten Arme können dann auf einfache Weise entfernt werden. Dies kann entweder durch Auskuppeln oder aber durch Abschneiden der Arme nahe ihrer Halterung erfolgen.

Eine abgewandelte zweckmäßige Ausführungsform der Erfindung kann darin bestehen, daß die Elektrodenarme aus elektrisch leitender Silikonfolie bestehen und als großflächige Segmente ausgebildet sind. Dabei können die Elektrodenarme als Kreissegmente ausgebildet sein und in Gebrauchsstellung gemeinsam etwa eine Kreisfläche überdecken, wobei zwischen jedem Segment eine schmale Lücke oder Isolierung vorgesehen ist. Dies ergibt wiederum einen Elektrodenkopf hoher Flexibilität bei gleichzeitig großflächiger Beaufschlagung des Herzens, so daß mit relativ geringen Energien bei dennoch kaum oder nicht eingeschränkter Bewegungsfreiheit des Herzens gearbeitet werden kann.

Eine weitere Abwandlung der Erfindung kann darin bestehen, daß die Elektrodenarme in eine fächer- oder schirmförmige Folie eingebettet sind oder zu einer solchen schirm- oder fächeförmigen Folie vereinigt sind, die entlang etwa radialer Knicklinien zusammenfaltbar ist. Dies ermöglicht vor allem eine temporäre Anwendung der Elektrode, die beim Lösen und Herausziehen aus dem Thorax selbsttäig zusammengefaltet werden kann.

Für die vorbeschriebenen Ausführungsbeispiele ist es besonders zweckmäßig, wenn die Zuleitung zum Elektrodenkopf mehrere Einzelleiter aufweist, deren jeder jeweils einzeln mit einem Elektrodenarm verbunden ist, wobei diese Einzelleiter zum Sensen elektrisch getrennt und zum Defibrillieren parallel geschaltet benutzbar sind. Das ist vor allem deshalb vorteilhaft, weil durch das Sensen zunächst einmal eindeutig festgestellt werden kann und muß, ob tatsächlich ein Kammerflimmern vorliegt. Hierbei können die entsprechenden Signale durch die vorbeschriebene Maßnahme von mehreren Stellen am Herzen aufgenommen und verglichen werden, um ganz sicher zu sein, daß das Herz flimmert. Zum Defibrillieren werden dann die Elektroden jeder Herzseite parallel geschaltet.

Insgesamt ergibt sich vor allem bei Kombination einzelner oder mehrerer der vorbeschriebenen Maßnahmen und Merkmale eine Defibrillations-Elektrode, die das Herz großflächig beaufschlagt, dennoch aber die Herzbewegung nicht oder nur sehr wenig beeinträchtigt.

Nachstehend ist die Erfindung mit ihren ihr als wesentlich zugehörenden Einzelheiten anhand der Zeichnung noch näher beschrieben. Es zeigt in schematisierter Darstellung :
Fig. 1 das Ende einer Defibrillations-Elektrode und insbesondere den dort angeordneten Elektrodenkopf mit mehreren sternförmig angeordneten Elektrodenarmen,
Fig. 2 ein Herz, an welchem auf der sichtbaren Seite drei Elektrodenköpfe etwa gemäß Fig. 1 einer Defibrillations-Elektrode angreifen,
Fig. 3 die Befestigung einer Defibrillations-Elektrode und ihres Elektrodenkopfes am Herzen mittels einer Einschraubwendel.
Fig. 4 einen Schnitt durch das Herzgewebe nach dem Befestigen eines erfindungsgemäßen Elektrodenkopfes nach dem Verankern der Elektrodenarme,
Fig. 5 eine abgewandelte Ausführungsform eines Elektrodenkopfes, bei welchem die einzelnen Elektrodenarme mit einer äußeren Isolierung versehen sind,
Fig. 6 in vergrößertem Maßstab eine Seitenansicht eines Elektrodenarmes des Elektrodenkopfes gemäß Fig. 5,
Fig. 7 einen Elektrodenkopf, bei welchem die Elektrodenarme eine abgewandelte Verankerung mit Hilfe eines hakenförmigen Spornes aufweisen,
Fig. 8 eine Seitenansicht des Endes eines Elektrodenarmes gemäß Fig. 7,
Fig. 9 einen abgewandelten Elektrodenkopf, bei welchem alle Arme von einem gemeinsamen Draconnetz zusammengefaßt sind,
Fig. 10 einen Elektrodenkopf, bei welchem die Elektrodenarme von elektrisch leitenden Foliensegmenten gebildet sind, die gemeinsam etwa eine Kreisfläche bedecken,
Fig. 11 einen Elektrodenkopf, bei welchem die Elektrodenarme von einer fächerartig zusammenfaltbaren Folie verbunden sind, in geöffneter Position sowie
Fig. 12 den fächerartigen Elektrodenkopf gemäß Fig. 11 in zusammengefalteter Lage beispielsweise beim Herausziehen aus dem Thorax.

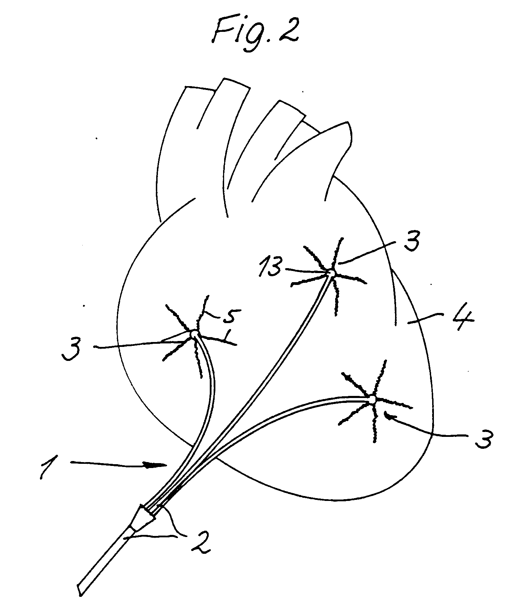

Eine in der Zeichnung jeweils nur teilsweise dargestellte, im ganzen mit 1 bezeichnete Defibrillations-Elektrode weist eine Zuleitung 2 und einen im ganzen mit 3 bezeichneten biegsamen vergrößerten Elektrodenkopf zum großflächigen Anlegen an der Außenseite des Herzens 4 (Fig. 2) auf. Die Zuleitung 2 führt zu einem nicht näher dargestellten Defibrillator oder Impulsgenerator, der für eine Notfallmaßnahme außerhalb des Körpers oder aber auch für dauergeschädigte Patienten implantiert sein kann. In diesem Falle ist die Defibrillations-Elektrode 1 ebenfalls implantiert.

In allen Ausführungsbeispielen erkennt man, daß der Elektrodenkopf 3 mehrere einzelne Elektrodenarme 5 aufweist, die in Gebrauchsstellung gemäß Fig. 2 stern- oder strahlenförmig auseinanderlaufen und einzeln an der Außenseite des Herzens 4 befestigbar sind. Die Zeichnungen zeigen dabei Beispiele von Elektrodenköpfen 3, die jeweils fünf Elektrodenarme 5 haben. Es ist jedoch auch eine andere Anzahl von Elektrodenarmen denkbar, je nachdem, an welcher Stelle des Herzens die Elektrode 1 angreifen soll und welche Platzverhältnisse dort herrschen.

Die im einzelnen noch zu beschreibenden Befestigungselemente für die Elektrodenarme 5 sind dabei jeweils an deren freiem Ende angeordnet und entweder als Zug- oder als Spannelemente ausgebildet.

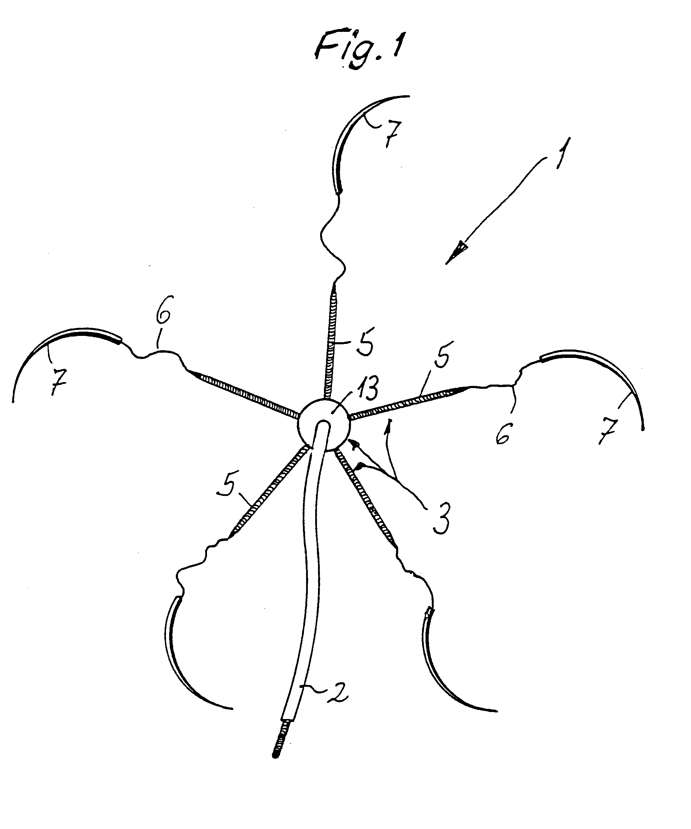

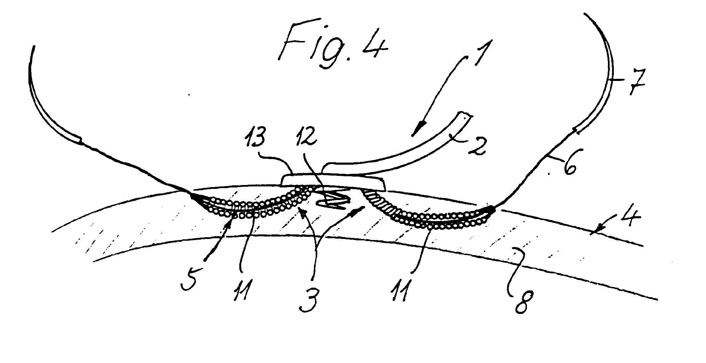

In den Ausführungsbeispielen nach Fig. 1 bis 4, 5, 6, 9, 10 und 11 sind am freien Ende eines jeden Elektrodenarmes 5 ein vorzugsweise elektrisch nicht leitender Faden 6 und an dessen freiem Ende eine chirurgische Nadel 7 zum Einziehen des Fadens 6 und des Elektrodenarmes 5 in das Herzgewebe 8 vorgesehen. Fig. 4 zeigt dabei die Elektrodenarme 5 nach deren Veankerung, d. h. nachdem die Nadel 7, der Faden 6 und schließlich auch der Elektrodenarm 5 in das Herzgewebe eingezogen wurden, wonach Nadel und Faden wieder daraus austreten und abgeschnitten werden können.

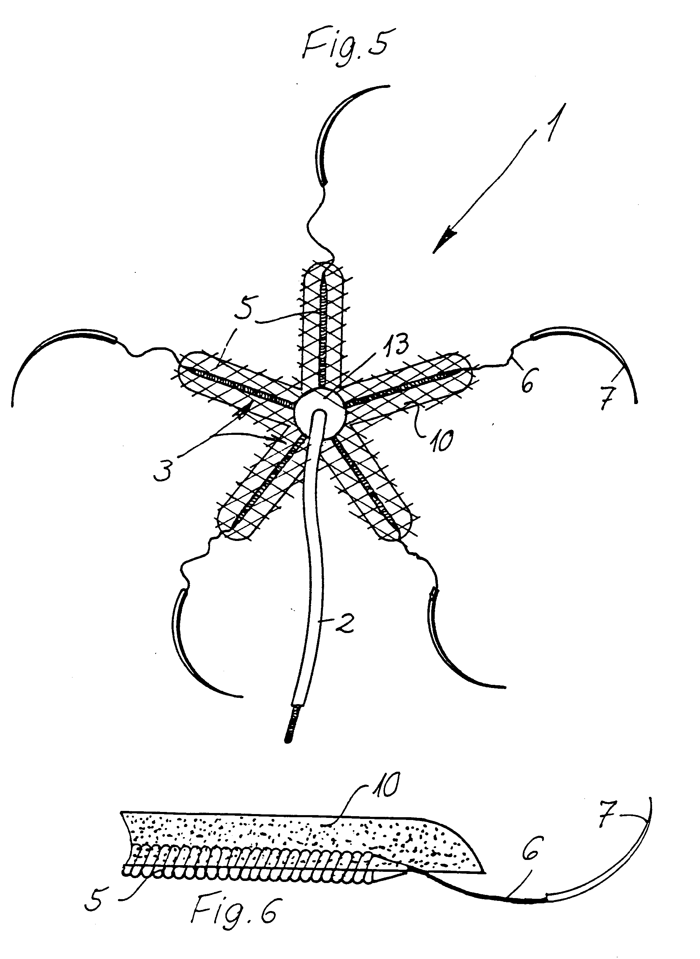

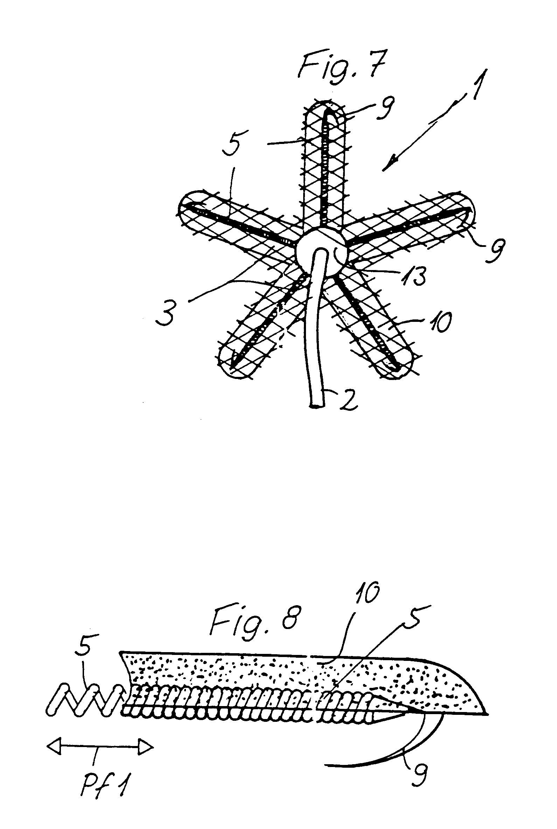

Eine andere Befestigungsmöglichkeit ist anhand der Figuren 7 und 8 angedeutet. In diesem Falle greift jeweils am Ende des Elektrodenarmes 5 ein quer zu dessen Erstreckung gegen das Innere des Herzens 4 gerichteter Sporn oder Haken 9 an. Durch den Doppelpfeil Pf 1 in Fig. 8 ist angedeutet, daß dabei der als aus blankem Draht bestehende Wendel ausgebildete Elektrodenarm 5, der auch eine Mehrfachwendel, beispielsweise eine Vierfachwendel sein kann, aufgrund dieser wendelförmigen Ausbildung etwa federartig auseinandergezogen werden kann, so daß nach Einstechen des Spornes oder Hakens 9 in das Herzgewebe 8 dieser selbsttätig tiefer eingezogen wird. Da in diesem Fall der Elektrodenarm 5 an der Außenseite des Herzens zu liegen kommt, also nicht wie bei Fig. 4 unter der Oberfläche des Herzens angeordnet wird, ist die Außenseite dieses Elektrodenarmes 5 mit einer Isolierung 10 versehen, die den Elektrodenarm 5 gegen den übrigen Körper isoliert. In den Figuren 5 und 6 erkennt man einen Elektrodenkopf 3 mit Elektrodenarmen 5, die zwar mit Hilfe einer Nadel 7 und eines Fadens 6 befestigt werden, aber ebenfalls an der Außenseite des Herzens 4 zu liegen kommen und nicht in die Herzwand eingezogen werden und deshalb ebenfalls eine derartige äußere Isolierung 10 haben.

In Fig. 4 erkennt man, daß die Elektrodenarm 5 bildende Wendel ein im wesentlichen undehnbares Sicherheitsband 11 zur Begrenzung oder Unterbindung einer Dehnung der Wendel in ihrer Längsrichtung aufweisen kann, was dann zweckmäßig ist, wenn die dieser wendelförmige Elektrodenarm in das Herzgewebe 8 eingezogen wird. Dabei kann dieses Sicherheitsband 11 aus Metall oder einem Faden aus chirurgischem od. dgl. körperverträglichem Nahtmaterial bestehen.

Während also bei der Elektrode gemäß Fig. 6 oder 8 eine gewisse Dehnung bei der Fixierung erwünscht oder zumindest unschädlich ist, weil sie auf der Außenseite des Herzens 4 fixiert wird, soll diese Dehnung dann unterbleiben, wenn der Elektrodenarm 5 in das Gewebe hineingezogen wird.

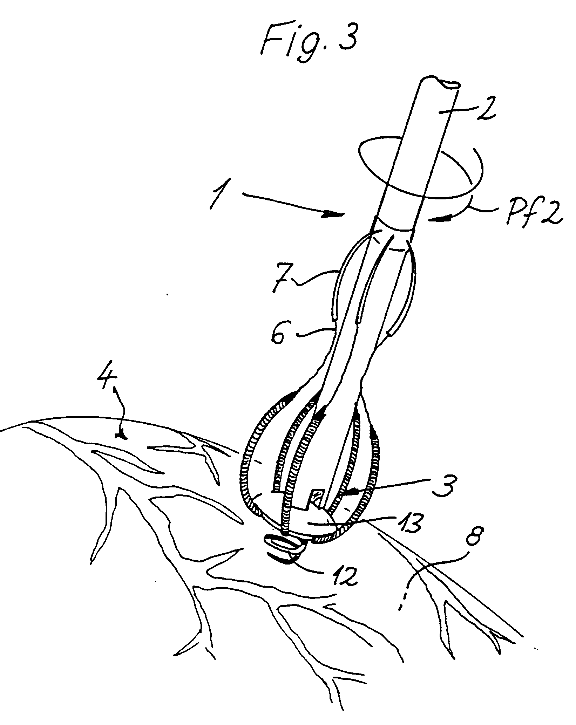

Insbesondere in den Figuren 3 und 4 erkennt man eine besonders zweckmäßige Ausgestaltung des Elektrodenkopfes 3, indem am gemeinsamen Ausgangspunkt der Elektrodenarme 5 eine die Zuleitung 2 etwa fortsetzende, insbesondere wendelförmige Schraube 12 zum Befestigen der Elektrode 1 am Herzen 4 vorgesehen ist. In Fig. 3 ist durch den schraubenförmigen Pfeil Pf 2 angedeutet, wie mit Hilfe dieser wendelförmigen Schraube 12 die gesamte Elektrode 1 durch eine Drehbewegung um die Achse der Zuleitung 2 zunächst an dem zentralen Punkt des Elektrodenkopfes 3 verankert werden kann. Die Elektrodenzuleitung 2 mündet oder endet dabei auf der Oberseite einer Halteplatte 13, von deren Unterseite die Einschraubwendel 12 und die Elektrodenarme 5 ausgehen. Dabei erkennt man in Fig. 3, daß die Elektrodenarme 5 gegenüber ihrer Halterung und der Halteplatte 13 schwenkbar sind und vor dem Anlegen der Elektrode 1 sowie auch während deren Einschrauben am Herzen 4 mit ihren freien Ende an der Zuleitung 2 der Elektrode 1 anliegen können, so daß sie den Einschraubvorgang nicht behindern.

Die erfindungsgemäße Ausgestaltung des Elektrodenkopfes 3 erlaubt also, die Vorteile einer Einschraubwendel 12 nutzbar zu machen. Gleichzeitig können die schwenkbaren Elektrodenarme 5 anschließend so an der Herzoberfläche befestigt werden, wie es aus anatomischen Gründen zweckmäßig ist, wobei sie keineswegs in regelmäßigem Winkelabstand zueinander, sondern unter Umständen auch mit unterschiedlichen Winkelabständen befestigt werden können. Gegenüber einer großflächigen aber im wesentlichen in ihrer Form festen Elektrode ergeben sich also eine Vielzahl von Anpassungsmöglichkeiten.

Statt der Einschraubwendel 12 könnte auch eine andere Befestigung zum Beispiel durch Annähen, mittels Haltehaken, - ähnlich den Haken 9 - mittels einer oder mehrerer Nadeln, mit Hilfe einer Klammer oder Klemme usw. erfolgen Die Elektrodenarme 5 sind von ihrer Halterung 13 abtrennbar, so daß gegebenenfalls auch weniger Arme 5 Verwendung finden können, falls dies aus anatomischen oder sonstigen Platzgründen zweckmäßig ist.

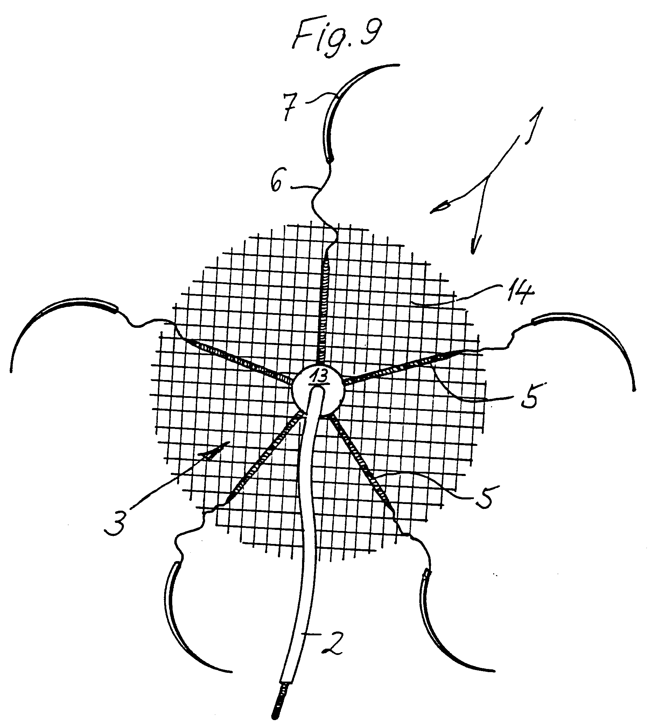

In Fig. 9 ist ein Ausführungsbeispiel dargestellt, bei welchem die Elektrodenarme 5 mit einem Draconnetz 14 verbunden sind, welches am Herzen annähbar ist. Ein solches Netz ist relativ flexibel und kann deshalb im Zusammenwirken mit den ebenfalls flexibeln, biegsamen und anpaßbaren Elektrodenarmen 5 ebenfalls eine gute und zusätzliche Befestigung zu den in Fig. 9 außerdem erkennbaren Nadeln 7 und Fäden 6 ergeben.

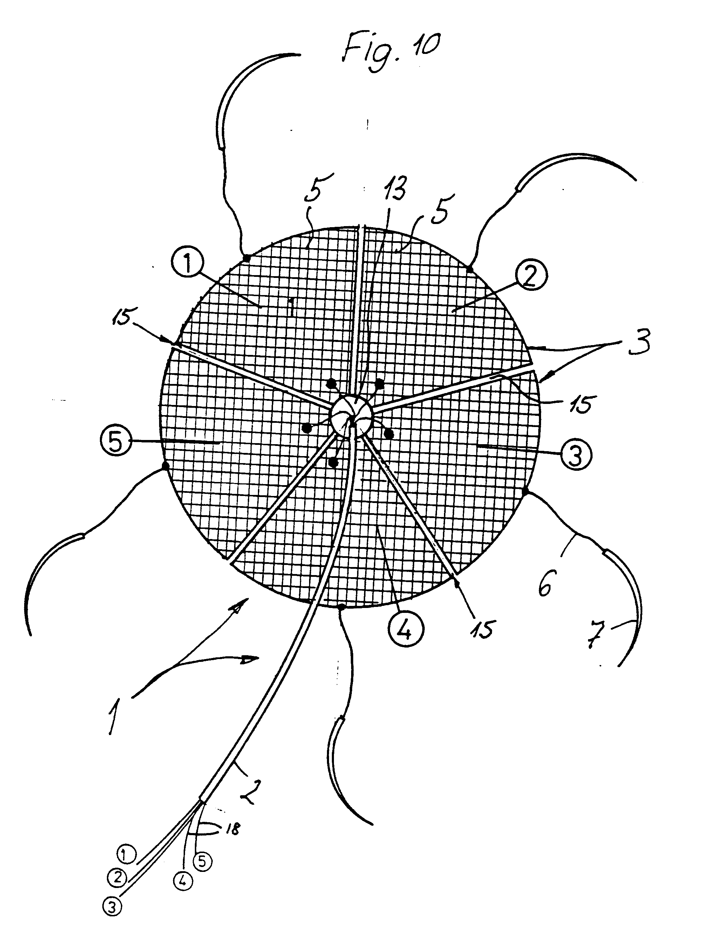

In Fig. 10 ist ein Ausführungsbeispiel dargestellt, bei welchem die Elektrodenarme 5 aus elektrisch leitender Silikonfolie bestehen und als großflächige Segmente ausgebildet sind. Dabei sind die Elektrodenarme 5 in diesem Falle als Kreissegmente ausgebildet, die in Gebrauchsstellung gemäß Fig. 10 gemeinsam etwa eine Kreisfläche überdecken, wobei man zwischen jedem Segment eine schmale Lücke 15 oder Isolierung erkennt. Bei dieser Ausführungsform wird also der Gedanke der einzelnen Elektrodenarme 5 damit kombiniert, dennoch eine möglichst großflächige und flächendeckende Beaufschlagung des Herzens 4 zu erreichen.

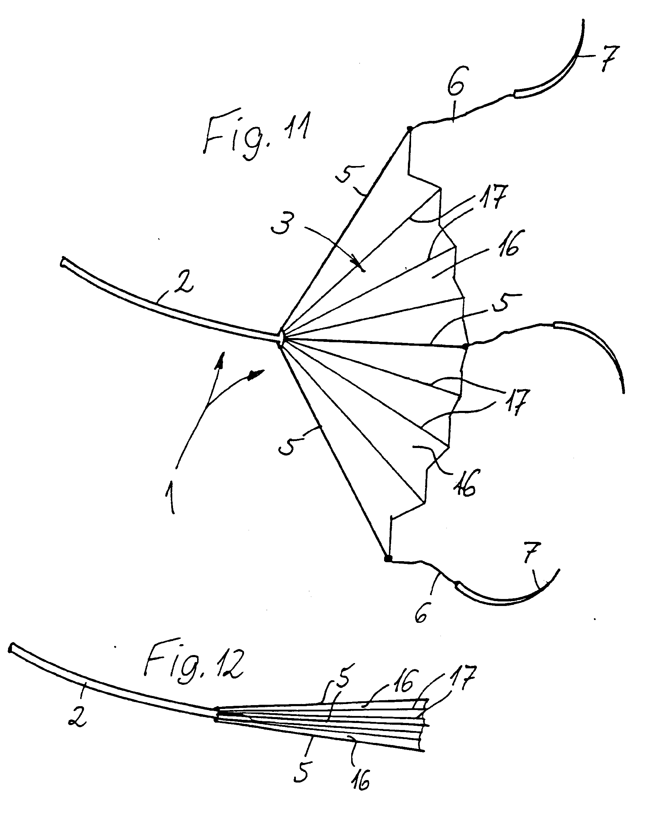

In den Figuren 11 und 12 ist eine Ausführungsform dargestellt, die sich vor allem für die kurzzeitige Anbringung am Herzen und eine Stimulation von außen her eignet. Die Elektrodenarme 5 sind dabei in einer fächer- oder schirmförmige Folie 16 eingebettet bzw. zu einer solchen Folie vereinigt, die entlang etwa radialer Knicklinien 17 zusammenfaltbar ist. Die zusammengefaltete Position erkennt man in Fig. 12 und dadurch wird ganz deutlich, daß ein derartig-gestalteter Elektrodenkopf 3 sehr einfach aus einer Körperöffnung wieder herausgezogen werden kann.

In Fig. 10 erkennt man außerdem, daß die Zuleitung 2 mehrere Einzelleiter 18 aufweist, die miht jeweils einem Elektrodenarm 5 verbunden sind und zum Sensen elektrisch getrennt und zum Defibrillieren parallel geschaltet benutzbar sind.

Die erfindungsgemäße Elektrode und insbesondere der in mehreren Ausführungsbeispielen vorstehend beschriebene Elektrodenkopf 3 kann in vorteilhafter Weise neben dem beschriebenen Einsatz bei der Defibrillation auch zur normalen Stimulation des Herzens verwendet werden, wenn ein entsprechender Stimulator oder Generator damit verbunden wird.

## Patentansprüche

1. Defibrillations-Elektrode (1) mit einem biegsamen vergrößerten Elektrodenkopf (3) zum großflächigen Anlegen an beliebiger Stelle der Außenseite des Herzens und mit einer Zuleitung (2) zur Verbindung des Elektrodenkopfes mit einem Defibrillator oder Impulsgenerator, dadurch gekennzeichnet, daß der Elektrodenkopf (3) mehrere einzelne Elektrodenarme (5) aufweist, die in Gebrauchsstellung vom Elektrodenkopf aus etwa stern- oder strahlenförmig auseinanderlaufen, einzeln an der Herzaußenseite befestigbar und allseitig schwenkbar sind

2. Elektrode nach Anspruch 1, dadurch gekennzeichnet, daß die Befestigungselemente für die Elektrodenarme (5) an deren freiem Ende etwa in Verlängerung dieser Arme angeordnet und als Zug- oder Spannelemente ausgebildet sind.

3. Elektrode nach Anspruch 1 oder 2, dadurch gekennzeichnet, daß am freien Ende eines jeden Elektrodenarmes (5) ein vorzugsweise elektrisch nicht leitender Faden (6) und an dessen freiem Ende eine chirurgische Nadel (7) zum Einziehen des Fadens (6) und/oder des Elektrodenarmes (5) in das Herzgewebe (8) vorgesehen sind.

4. Elektrode nach einem der Ansprüche 1 bis 3, dadurch gekennzeichnet, daß am Ende des Elektrodenarmes (5) ein quer zu dessen Erstreckung gegen das Innere des Herzens (4) gerichteter Sporn oder Halter (9) angreift.

5. Elektrode nach einem der Ansprüche 1 bis 4, dadurch gekennzeichnet, daß der Elektrodenarm (5) als aus blankem Draht bestehende Wendel ausgebildet ist.

6. Elektrode nach einem der Ansprüche 1 bis 5, dadurch gekennzeichnet, daß der Elektrodenarm als Mehrfachwendel, beispielsweise als Vierfachwendel ausgebildet ist.

7. Elektrode nach einem der Ansprüche 1 bis 6, dadurch gekennzeichnet, daß die den Elektrodenarm (5) bildende Wendel ein im wesentlichen undehnbares Sicherheitsband (11) zur Begrenzung oder Unterbindung einer Dehnung der Wendel in ihrer Längsrichtung aufweist.

8. Elektrode nach einem der Ansprüche 1 bis 7, dadurch gekennzeichnet, daß das Sicherheitsband aus Metall und/ oder einem Faden aus chirugischem od. dgl. körperverträglichem Nahtmaterial besteht.

9. Elektrode nach einem der Ansprüche 1 bis 8, dadurch gekennzeichnet, daß der Elektrodenkopf (3) zusätzlich zu seiner Befestigung mittels der Elektrodenarme (5) auch an dem gemeinsamen Ausgangspunkt der Elektrodenarme (5) an der Herzaußenseite befestigbar oder verankerbar ist, insbesondere eine Befestigungsstelle oder Befestigungsvorrichtung, vorzugsweise eine zum Beispiel wendelförmige Schraube (12), Klammer od. dgl. aufweist.

10. Elektrode nach einem der Anspruche 1 bis 9, dadurch gekennzeichnet, daß die Elektrodenzuleitung (2) - vorzugsweise an der Oberseite - an einer Halteplatte (13) mündet oder endet, von deren Unterseite eine Einschraubwendel (12) od. dgl. und/oder die Elektrodenarme (5) ausgehen.

11. Elektrode nach einem der Ansprüche 1 bis 10, dadurch gekennzeichnet, daß die Elektrodenarme (5) gegenüber ihrer Halterung schwenkbar sind und vor dem Anlegen der Elektrode mit ihren freien Enden an der Zuleitung (2) der Elektrode (1) anliegen.

12. Elektrode nach einem der vorstehenden Ansprüche, dadurch gekennzeichnet, daß die Elektrodenarme (5) mit einem Draconnetz (14) verbunden sind, welches am Herzen annähbar ist

13. Elektrode nach einem derAnsprüche 1 bis 12, dadurch gekennzeichnet, daß die Elektrodenarme (5) von ihrer Halterung (13) abtrennbar sind.

14. Elektrode nach einem der Ansprüche 1 bis 13, dadurch gekennzeichnet, daß die Elektrodenarme (5) aus elektrisch leitender Silikonfoliebestehen und als großflächige Segmente ausgebildet sind.

15. Elektrode nach einem derAnsprüche 1 bis 14, dadurch gekennzeichnet, daß die Elektrodenarme (5) als Kreissegmente ausgebildet sind und in Gebrauchsstellung gemeinsam etwa eine Kreisfläche überdecken, wobei zwischen jedem Segment eine schmale Lücke (15) oder Isolierung vorgesehen ist.

16. Elektrode nach einem der vorstehenden Ansprüche, dadurch gekennzeichnet, daß die Elektrodenarme (5) in eine fächer- oder schirmförmige Folie (16) eingebettet sind oder zu einer solchen schrim- oder fächerförmigen Folie vereinigt sind, die entlang etwa radialer Knicklinien (17) zusammenfaltbar ist.

17. Elektrode nach einem der vorstehenden Ansprüche, dadurch gekennzeichnet, daß die Zuleitung (2) mehrere Einzelleiter (18) aufweist, die mit jeweils einem Elektrodenarm (5) verbunden sind und zum Sensen elektrisch getrennt und zum Defibrillieren parallel geschaltet benutzbar sind.

18. Elektrode nach einem der Ansprüche 1 bis 17, dadurch gekennzeichnet, daß auf der Herzaußenseite anbringbare Elektrodenarme (5) eine äußere dem Herzen (4) abgewandte Isolierung (10) haben.

## Claims

1. A defibrillation electrode (1) including a pliant, enlarged electrode head (3) for being applied to a large area at any location of the exterior of the heart, and further including a lead (2) for connecting the electrode head to a defibrillator or pulse generator, characterized in that the electrode head (3) has a plurality of individual electrode arms (5) which in the position for use diverge from the electrode head in an approximately star- like or radial manner, are individually attachable to the exterior of the heart and are orientable in all directions.

2. The electrode as claimed in claim 1, characterized in that the members for attaching the electrode arms (5) are disposed at the free end of the latter, approximately in extension of said arms, and take the form of drawing or tensioning members.

3. The electrode as claimed in claim 1 or claim 2, characterized in that the free end of each electrode arm (5) is provided with a thread (6), preferably one which is not electrically conductive, and the free end of the latter is provided with a surgical needle (7) for drawing the thread (6) and/orthe electrode arm (5) into the tissue of the heart.

4. The electrode as claimed in any one of claims 1 to 3, characterized in that the end of the electrode arm (5) is engaged by a spur or retaining means (9) which is directed transversely to the direction in which the electrode arm extends, towards the inside of the heart (4).

5. The electrode as claimed in any one of claims 1 to 4, characterized in that the electrode arms (5) takes the form of a coil consisting of bare wire.

6. The electrode as claimed in any one of claims 1 to 5, characterized in that the electrode arm takes the form of a multiple coil, e.g. a quadruple coil.

7. The electrode as claimed in any one of claims 1 to 6, characterized in that the coil composing the electrode arm (5) has an essentially inextensible safety band (11) to restrict or prevent the coil from being extended in its longitudinal direction.

8. The electrode as claimed in any one of claims 1 to 7, characterized in that the safety band consists of metal and/or thread of surgical or suchlike suture material compatible with the body.

9. The electrode as claimed in any one of claims 1 to 8, characterized in that in addition to being attached by means of the electrode arms (5), the electrode head (3) is also adapted to be attached or anchored to the exterior of the heart at the common starting point of the electrode arms (5), in particular has a place or means for attachment, preferably a screw (12), e.g. a helical one, clip or the like.

10. The electrode as claimed in any one of claims 1 to 9, characterized in that the electrode lead (2) ends at a holding plate (13) - preferably at the upper side - , a screw-in spiral (12) orthe like and/or the electrode arms departing from the underside of said holding plate.

11. The electrode as claimed in any one of claims 1 to 10, characterized in that the electrode arms (5) are swingable relative to their holder and restwith their free ends against the lead (2) of the electrode (1) before the electrode is applied.

12. The electrode as claimed in any one of the preceding claims, characterized in that the electrode arms (5) are connected to a dracon net (14) which is adapted to be sutured to the heart.

13. The electrode as claimed in any one of claims 1 to 12, characterized in thatthe electrode arms (5) are detachable from their holder (13).

14. The electrode as claimed in any one of claims 1 to 13, characterized in that the electrode arms (5) consists of electrically conductive silicone foil and take the form of large segments.

15. The electrode as claimed in any one of claims 1 to 14, characterized in thatthe electrode arms (5) take the form of segments of a circle and in the position for use together cover approximately a circular area, a narrow gap (15) or insulation being provided between each segment.

16. The electrode as claimed in any one of the preceding claims, characterized in that the electrode arms (5) are embedded in a fan-shaped or umbrella-shaped foil (16) or are combined to form such an umbrella-shaped or fan-shaped foil, which is foldable along approximately radial crease lines (17).

17. The electrode as claimed in any one of the preceding claims, characterized in that the lead (2) has a plurality of single conductors (18) which are connected in each case to an electrode arm (5) and are usable in an electrically separate condition for sensing and in a condition connected in parallel for defibrillating.

18. The electrode as claimed in any one of claims 1 to 17, characterized in that electrode arms (5) which are adapted to be applied to the exterior of the heart have an outer insulation (10) averted from the heart (4).

## Revendications

1. Electrode de défibrillation (1) comportant une tête d'électrode (3) souple, agrandie, destinée à être appliquée sur une grande surface à un endroit quelconque de la face extérieure du coeur, et comportant une ligne électrique (2), reliant la tête d'électrode à un défibrillateur ou à un générateur d'impulsions, caractérisée en ce que la tête d'électrode (3) comporte plusieurs bras d'électrode (5) qui, en position d'utilisation, s'écartent les uns des autres en étoile ou en rayons à partir de la tête d'électrode, peuvent être fixés séparément sur la face extérieure du coeur, et sont pivotants en tous sens.

2. Electrode selon la revendication 1, caractérisée en ce que les éléments de fixation des bras d'électrode (5) sont placés à leur extrémité libre, sensiblement dans le prolongement de ces bras, et sont conformés en éléments de traction ou de tension.

3. Electrode selon la revendication 1 ou 2, caractérisée en ce qu'à l'extrémité libre de chaque bras d'électrode (5) il est prévu un fil (6) de préférence non conducteur de l'électricité, et à l'extrémité libre de celui-ci une aiguille chirurgicale (7) pour introduire le fil (6) et/ou le bras d'électrode (5) dans le tissu cardiaque (8).

4. Electrode selon l'une des revendications 1 à 3, caractérisée en ce qu'un éperon ou un crochet (9) dirigé vers l'intérieur du coeur (4) et s'étendant perpendiculairement à la direction longitudinale du bras d'électrode (5), est prévu à l'extrémité de celui-ci.

5. Electrode selon l'une des revendications 1 à 4, caractérisée en ce que le bras d'électrode (5) est une hélice en fil nu.

6. Electrode selon l'une des revendications 1 à 5, caractérisée en ce que le bras d'électrode est une hélice multiple, par exemple une hélice quadruple.

7. Electrode selon l'une des revendications 1 à 6, caractérisée en ce que l'hélice formant le bras d'électrode présente un ruban de sécurité (11) pratiquement inextensible, destiné à limiter ou à empêcher un allongement de l'hélice dans sa direction longitudinale.

8. Electrode selon l'une des revendications 1 à 7, caractérisée en ce que le ruban de sécurité est réalisé dans un métal et/ou dans un fil à coudre chirurgical pouvant être toléré par le corps, ou une matière similaire.

9. Electrode selon l'une des revendications 1 à 8, caractérisée en ce que la tête d'électrode (3) peut être fixée ou ancrée, en plus de sa fixation au moyen des bras d'électrode (5), au point de départ commun des bras d'électrode (5), sur la face extérieure du coeur, et présente en particulier un point de fixation ou un dispositif de fixation, de préférence une vis (12) par exemple hélicoïdale, une agrafe ou similaire.

10. Electrode selon l'une des revendications 1 à 9, caractérisée en ce que la ligne électrique de l'électrode (2) arrive ou se termine - de préférence sur la face supérieure - sur une plaque de fixation (13), une hélice de vissage (12) ou similaire et/ou les bras d'électrode (5) partant de sa face inférieure.

11. Electrode selon l'une des revendications 1 à 10, caractérisée en ce que les bras d'électrode peuvent pivoter par rapport à leur support, et s'appliquent par leurs extrémités libres contre la ligne électrique (2) de l'électrode (1), avant application de l'électrode.

12. Electrode selon l'une des revendications ci-dessus, caractérisée en ce que les bras d'électrode (5) sont assemblés par un filet en Dracon (14) qui peut être cousu sur le coeur.

13. Electrode selon l'une des revendications 1 à 12, caractérisée en ce que les bras d'électrode (5) peuvent être séparés de leur support (13).

14. Electrode selon l'une des revendications 1 à 13, caractérisée en ce que les bras d'électrode sont réalisés dans une feuille silicone conductrice de l'électricité, et sont des segments de grande surface.

15. Electrode selon l'une des revendications 1 à 14, caractérisée en ce que les bras d'électrode (5) sont des segments circulaires, et recouvrent ensemble, en position d'utilisation, une surface sensiblement circulaire, une fente (15) étroite ou un isolement étant prévu entre chaque segment.

16. Electrode selon l'une des revendications ci-dessus, caractérisée en ce que les bras d'électrode (5) sont noyés dans une feuille (16) en forme d'éventail ou de parapluie, ou sont réunis pour former une telle feuille en parapluie ou en éventail qui peut être repliée le long de lignes de pliage (17) à peu près radiales.

17. Electrode selon l'une des revendications ci-dessus, caractérisée en ce que la ligne électrique (2) comporte plusieurs conducteurs (18) qui sont reliés chacun à un bras d'électrode (5) et qui sont électriquement séparés pour capter, et montés en parallèle pour la défibrillation.

18. Electrode selon l'une des revendications 1 à 17, caractérisée en ce que les bras d'électrode (5) à appliquer sur la face extérieure du coeur, ont une isolation (10) extérieure, à l'opposé du coeur (4).
